# EUROPEAN PATENT SPECIFICATION

(11) **EP 2 026 763 B1**
(45) Date of publication and mention of the grant of the patent: **29.06.2011**
(21) Application number: 07747448.4
(22) Date of filing: 16.05.2007
(51) Int. Cl.: A61K 9/00, A61K 31/715, A61K 31/718, A61K 31/722, A61K 31/724, A61K 31/731, A61K 31/732, A61K 31/738, A61K 31/736

(54) **A METHOD TO REDUCE THE SYMPTOMS OF HEARTBURN AND GASTRO-OESOPHAGEAL REFLUX DISEASE (GERD) BY SPECIFIC POLYSACCHARIDES**
VERFAHREN ZUR REDUZIERUNG DER SYMPTOME VON SODBRENNEN UND GASTROÖSOPHAGEALER REFLUX-KRANKHEIT (GERD) DURCH SPEZIFISCHE POLYSACCHARIDE
PROCÉDÉ DE RÉDUCTION DES SYMPTÔMES DE PYROSIS ET DE REFLUX GASTRO- SOPHAGIEN (RGO) PAR DES POLYSACCHARIDES SPÉCIFIQUES

(30) Priority: 16.05.2006 EP 06076056; 30.06.2006 US 806313 P
(43) Date of publication of application: 25.02.2009
(73) Proprietor: Frutarom Netherlands B.V., 3905 PE Veenendaal (NL)
(72) Inventor: VERBRUGGEN, Maria Anna, 6871 DH Renkum (NL); KELLY, Calvin T., Regina, Saskatchewan S4P 3R8 (CA); SUDOM, Blaine G., Avonlea, Saskatchewan S0H 0C0 (CA)
(74) Representative: Hatzmann, Martin
(86) International application number: PCT/NL2007/050224
(87) International publication number: WO 2007/133082

(56) References cited:
- EP-A1- 0 059 221
- WO-A2-01/66119
- WO-A2-03/068246
- GB-A- 2 324 725
- GB-A- 2 349 570
- BATCHELOR H K ET AL: "An in vitro mucosal model for prediction of the bioadhesion of alginate solutions to the oesophagus" INTERNATIONAL JOURNAL OF PHARMACEUTICS, AMSTERDAM, NL, vol. 238, no. 1-2, 15 May 2002 (2002-05-15), pages 123-132, XP002289236 ISSN: 0378-5173

## Description

The invention is directed to the use of a specific group of polysaccharides for reducing the symptoms of heartburn and gastro-oesophageal reflux disease.

The human stomach is a complex system which has different functions, such as to store food, to initiate the digestion of proteins, to kill bacteria and to move food into the small intestine as a pasty material called chyme.

When an individual consumes food, the swallowed food is passed through the oesophagus to the stomach by wavelike contractions known as peristalsis. The lumen of the terminal portion of the oesophagus is slightly narrowed because of a thickening of the circular muscle fibres in its wall: the lower oesophageal or gastro-oesophageal sphincter. After food passes into the stomach, constriction of the muscle fibre prevents the stomach contents from regurgitating into the oesophagus. Regurgitating would occur because of pressure differences on both sides as a result of respiratory movements.

The stomach can be divided into an upper fundus, a lower body and a distal pyloric region that ends in the pyloric sphincter that connects the stomach to the duodenum. The gastric glands are formed by a folded mucosa layer, secreting various products through the so called gastric pits into the gastric lumen. The gastric glands contain several types of cells secreting different products:
- Goblett cells (in fundus and pyloric region) secrete mucus
- Parietal cells (in fundus) secrete hydrochloric acid (HCl)
- Chief cells (in fundus and pyloric region) secrete pepsinogen
- Enterochromaffin-like (ECL) cells (in fundus) secrete histamine
- G-cells (in pyloric region) secrete the hormone gastrin
- D-cells (in fundus and pyloric region) secrete the hormone somatostatin

Regulation of gastric acid secretion can be divided into three phases. The 'cephalic phase' refers to control by the brain via the vagus nerve. Sight, smell, taste of food, or other conditioned stimuli (Pavlov effect) cause a stimulation of vagus nuclei in the brain. Activation of the vagus nerve stimulates secretion of pepsinogen, gastrin, and histamine. The secreted gastrin enhances histamine secretion. The secreted histamine activates the parietal cells to secrete HCl.

The arrival of food into the stomach stimulates the 'gastric phase'. The amount and preparation of the chyme are important stimulants for gastric secretion. In particular, partly digested polypeptides and amino acids stimulate secretion of pepsinogen and gastrin. Gastrin, in turn, stimulates the secretion of pepsinogen. Again, the gastrin enhances histamine production, which stimulates HCl production by the parietal cells. The amount of secreted acid is matched to the amount of protein digested by two feedback mechanisms: as more HCl and more pepsinogen are secreted, more short polypeptides and amino acids are released from the ingested proteins. Further secretion is stimulated. The secretion of gastrin, however, drops together with the pH of the gastric juice. Accordingly, the secretion of HCl declines. Further, upon a pH drop the D cells are stimulated to secrete somatostatin that inhibits the secretion of gastrin.

The final phase is called the 'intestinal phase' and refers to the inhibition of gastric activity when the food enters the small intestine. The drivers are (1) a neural reflex initiated by an increase in osmotic pressure as well as stretch of the duodenum, and (2) a chemical hormone enterogastrone secreted by the duodenum in response to fat.

In summary gastric acid secretion is a complex, continuous process controlled by multiple central (neural) and peripheral (endocrine and paracrine) factors. Each factor attributes to a common final physiological event, which is the secretion of H⁺ by parietal cells via the activation of H⁺/K⁺-ATPase, also called the proton pump.

The stomach protects itself from excess acid through several mechanisms. There is a constant confrontation in the stomach and upper small intestine between acid-pepsin aggression and mucosal defence. Usually the mucosa can withstand the acid-pepsin attack and remain healthy, i.e. a mucosal 'barrier' to back diffusion of acid is maintained. The cell membranes of the parietal and chief cells of the gastric mucosa are highly impermeable to the acid. Other protective mechanisms include a layer of alkaline mucus, containing bicarbonate, covering the gastric mucosa. The presence of intercellular tight junctions between adjacent epithelium cells prevent acid from leaking into the sub-mucosa. The rapid rate of cell division allows damaged cells to be replaced timely. The production of prostaglandins by the gastric mucosa provides a range of protective effects. Prostaglandins inhibit gastric acid secretion. They further enhance mucosal blood flow and stimulate the secretion of mucus and bicarbonate. However, an excess of production of acid or an intrinsic defect in the barrier functions in the mucosa can cause the defence mechanism to fail and ulcers to form. When these gastric barriers to self-digestion are broken down, acid can indeed leak through the mucosa into the sub-mucosa, causing direct damage and stimulating inflammation (gastritis).

Many people experience from time to time discomforts upon food consumption. This problem may occur when the lower oesophageal sphincter permits the acidic contents of the stomach to (re-)enter the oesophagus. This can create a burning sensation commonly called heartburn. It is estimated that 7-10 % of the US population experiences this on a daily basis. Gastro-oesophageal reflux disease (GERD) is most often also associated with inappropriate relaxation of the lower oesophageal sphincter. Acidic gastric content is allowed to flow into the oesophagus, and produce painful irritation and inflammation of the oesophageal mucosa. GERD can be reduced using drugs that increase the tone of the lower oesophageal sphincter, and mucosal irritation can be reduced by decreasing the concentration of acid in the stomach. This disorder involving the reflux of acidic gastric juice into the oesophagus is very common.

The term acid-peptic disorders encompass a variety of relatively specific medical conditions in which injury by gastric acid (and activated pepsin) is thought to play an important role. These disorders include GERD, benign peptic ulcers of the stomach and duodenum, ulcers secondary to the use of conventional non-steroidal anti-inflammatory drugs (NSAIDs), and ulcers due the rare Zollinger-Ellison syndrome (in response to extremely high levels of gastrin).

It appears that exposure of the involved tissue to acid is essential to the development of clinical symptoms in most instances of these diseases. Control of gastric acidity is therefore a cornerstone of therapy in these disorders, even though this approach may not address the fundamental patho-physiological process.

Current medicinal therapies for treating gastric acid mediated clinical conditions include the use of proton pump inhibitors, histamine H₂-receptor antagonists for inhibition of acid production, the use of prostaglandin analogues for inhibition of acid secretion, the use of sulphated polysaccharides to inhibit mucosal erosion and ulcerations, and the use of antacids to neutralise gastric acid (Katz Rev. Gastroenterol. Disord. 2003, 3(2), 59-69). The most commonly used agents are the proton pump inhibitors and the histamine H₂-receptor antagonists.

Regurgitation and vomiting is a common reaction to milk feeding in infants. Milk formulas enriched with water-soluble fibres, such as galactomannans, are a first-line measure in infants with frequent regurgitation and vomiting. Fabiani *et al.* showed the ingestion of the water-soluble fibre-enriched formula does not have any significant influence on the gastric emptying time of the infants (Fabiani et al. J. Pediatr. Gastroenterol Nutr. 2000, 31(3), 248-250). The water-soluble fibres are added to the milk in order to increase the viscosity of the milk. The increased viscosity of the food is believed to result in a reduction of regurgitation and vomiting. The fibres are administered in dissolved form as part of the food.

Harju *et al.* showed that the dietary fibre guar gum, which is composed of galactose and mannose, is helpful to patients with uncomplicated duodenal ulcer as a component in food (Harju et al. J. Parenter. Enteral. Nutr. 1985, 9(4), 496-500). The dietary fibre guar gum may be harmful to patients with an elevated stomach emptying time.

The British patent application 2 349 570 discloses compositions for treatment of disorders of the oesophagus, especially those associated with reflux such as dyspepsia, oesophagitis and gastritis. These compositions comprise at least three different components: (A) an alginate salt, (B) xanthan gum, carrageenan gum or a mixture of the two, and (C) a glucomannan, a galactomannan or a mixture of the two. It is noted that alginates are linear polysaccharides composed of mannuronic acid and guluronic acid. The publication only shows effects for liquid compositions, which should adhere to the oesophageal mucosa to become effective. No effects on acid reflux are shown. Since the shown adherence to the oesophageal mucosa does not last longer than 15 to 26 minutes (example 7) the effect is not expected to last long The compositions therefore appear to be intended as a replacement for anti-acid medicines, which are commonly used after the problems occur, rather than as a preventive treatment of gastric acid episodes. Furthermore, these alginate based products preferably contain a gas forming component in order to create a foam (raft) to float on top of the gastric content to prevent reflux.

The international patent application 01/66119 discloses a pharmaceutical composition for use in the healing of cells comprising an alginic acid or an alginate salt. The document expresses a preference for liquid dosages forms.

The British patent application 2 324 725 discloses a pharmaceutical composition suitable for forming a mucoadhesive lining in the gastrointestinal tract, again based on alginic acid or an alginate salt.

Object of the present invention is to provide a pharmaceutical preparation for reducing the symptoms of heartburn and/or gastro-oesophageal reflux disease which lacks one or more of the above-mentioned disadvantages.

This object can be met using a specific group of polysaccharides. Hence, in a first aspect the present invention is directed to a pharmaceutical preparation comprising an effective amount of a water-soluble branched or linear polysaccharide, wherein said polysaccharide has a hydration rate, measured by the viscosity development curve over time, reaching a plateau viscosity within at most 20 minutes as measured during a 120 min period in a 1 wt.% solution hydrated at 60°C at 500 rpm in a Rapid Visco-analyser, and wherein said polysaccharide maintains at least 80 % of its number average molecular weight when exposed to an aqueous solution with a pH of 2-3 at 37°C for at least 6 hours, and wherein the apparent viscosity of a 1 wt.% solution of said polysaccharide in water is at least 500 mPa·s (cps) at 20 °C and shear rates up to 60 s⁻¹ as measured by a Haake Rotoviscometer.

The inventors surprisingly found that the preparation according to the invention is particularly effective in the treatment of heartburn and/or gastro-oesophageal reflux as a food supplement apart from the food, particularly when consumed in dry form. The term treatment should be construed as to cover both therapeutic and prophylactic treatments.

Without wishing to be bound by theory, it is believed that the polysaccharide, once in the stomach, quickly takes up a large amount of water due to the high hydration rate of the polysaccharide. This results in a highly viscous solution which is thought to form a protective layer in the stomach and thus acts as an artificial mucosa barrier. Preferably, the polysaccharide of the present invention has a main chain which is composed for more than 50 wt.% of one or more units that are chosen from the group consisting of glucose, galactose, mannose, glucuronic acid, galacturonic acid and mannuronic acid. The units of the other 50 wt.% of the main chain can be a mixture of arbitrary sugar units. It is preferred that the polysaccharide is composed of not only acidic sugar units, but also comprises neutral sugar units, or is composed of only neutral sugar units. Although it is possible to use mixtures of different polysaccharides, it is preferred that the pharmaceutical preparation comprises polysaccharides having at least 50% in its main chain of sugar units chosen from the group consisting of glucose, galactose, mannose, glucuronic acid, galacturonic acid and mannuronic acid, only. Even more preferred is the situation wherein the pharmaceutical preparation comprises only one type of polysaccharide.

The polysaccharide may be branched or linear. In a preferred embodiment 50 wt.% of the units of the branches are chosen from the group consisting of glucose, galactose, mannose, glucuronic acid, galacturonic acid, and mannuronic acid. The degree of substitution of the polysaccharide can vary from zero to 1.3. In a preferred embodiment, the polysaccharide is a galactomannan. For galactomannans the degree of substitution is preferably between 0.25 and 1.1. A high degree of substitution in this case contributes to high hydration rate observed in galactomannans.

In accordance with the invention, the polysaccharide has a fast hydration rate, *viz*. the polysaccharide is able to take up a large amount of water in short time. Methods to measure the exact hydration rate in soluble fibres are difficult, and hardly available. Therefore, in accordance with the invention, the viscosity increase as a function of time is taken as a measure for the hydration rate.

In accordance with the invention, the viscosity increase as a function of time is determined as follows. A sample of soluble fiber is pre-wetted with ethanol. The ethanol/fiber ratio is 5 ml / 1g. Water is added to the pre-wetted fiber in a ratio of approximately 16 / 1. A Rapid Visco-analyser (RVA) is used for measuring the viscosity increase during a period of 120 minutes (at 60°, mixing speed 500 rpm). The sample is shaken vigorously before insertion into the RVA holder. Using this method, the polysaccharide reaches its peak viscosity within at most 20 minutes, and preferably within 15 minutes.

Furthermore, a 1 wt.% solution of the polysaccharide used in accordance with the invention in water at 20 °C gives rise to an apparent viscosity of at least 500 mPa·s (cps) measured using shear rates up to 60 s⁻¹ (Haake Rotoviscometer). It is preferred that the viscosity of a 1.5 wt.% solution of the polysaccharide in water at 20 °C is at least 1500 mPa·s (cps). The viscosities as mentioned in this application are measured by a Haake Rotoviscometer, unless otherwise indicated.

The polysaccharide of the invention should preferably be stable at low pH, so that it does not disintegrate under the acidic conditions of the stomach. In particular, this encompasses that the polysaccharide should preferably maintain at least 80 %, more preferably at least 90%, of its original number average molecular weight when exposed to an aqueous solution with a pH of 2-3 at 37 °C for at least 6 hours.

Preferred polysaccharides that can be comprised in the preparation of the present invention include galactomannans.

Polysaccharides to be used in accordance with the invention can for instance be obtained from fenugreek seed by methods as described in CA-A-2 206 157. In a highly preferred embodiment, a fraction from fenugreek seed is used. With reference to the procedure disclosed in CA-A-2 206 157, it is preferred that the soluble dietary fibre fraction, sometimes also referred to as fenugreek fibre, fenugreek soluble fibre or fenugreek galactomannan, is used.

The preparation according to the invention may further comprise a pharmaceutically acceptable excipient. The pharmaceutically acceptable excipient can for instance be chosen from the classes of diluents (such as sodium and calcium carbonates, sodium and calcium phosphates, and lactose), disintegrants (such as cornstarch), granulating agents, lubricants (such as magnesium stearate, stearic acid, and talc), binders (such as starch and gelatine), thickeners (such as paraffin; waxes, and petrolatum), flavouring agents, colouring agents, preservatives, fillers, sweeteners, antioxidants, coating materials. Combinations of these excipients are also possible.

The pharmaceutical preparation can be prepared using conventional pharmaceutical excipients and compounding techniques. In accordance with the invention, the pharmaceutical preparation preferably has a solid, unit dosage form. Examples of suitable unit dosage forms are coated tablets, capsules, coated pills. Powders, powder packets, granules, wafers, and the like, as well as liquid preparations should not be applied as these would cause high viscosity in the mouth and esophagus, rather than in the stomach. The material preferably is protected from early exposure to moisture as in a capsule. Alternatively tablets can be used, coated by sugar, compression or film coating to facilitate easy and quick swallowing and dissolution in the stomach. As an example materials like hydroxypropyl methylcellulose or hydroxypropyl cellulose can be used for this purpose.

Examples of solid carriers that may be used in the preparation of the invention include those materials usually employed in the manufacture of pills or tablets, such as lactose, starch, glucose, methylcellulose, magnesium stearate, dicalcium phosphate, mannitol and the like, thickeners such as tragacanth and methylcellulose USP, finely divided SiO₂, polyvinylpyrrolidone, magnesium stearate, and the like.

The pharmaceutical preparation may further have a coating or more than one coating layers, such as a fat coating. The preparation may also contain adjuvants (such as preserving, wetting, emulsifying, and dispensing agents), antimicrobial agents (such as parabens, chlorobutanol, phenol, and sorbic acid), isotonic agents (such as sugar or sodium chloride), absorption-prolonging agents (such as aluminium monostearate and gelatine), and absorption-enhancing agents.

The preparation according to the present invention is preferably to be administered orally. The compounds according to the invention will be generally provided in the form of tablets or capsules.

Pharmaceutically effective doses of the polysaccharide according to the invention that are applied in the unit dosage form may be ascertained by conventional methods. The specific dosage level required for any particular subject will depend on a number of factors, including severity of the heartburn or gastro-oesophageal reflux disease and the administration of other medicaments. Other medicaments that may be combined with the pharmaceutical preparation of the present application are in particular antacids, inhibitors of proton pumps, histamine H₂-receptor antagonists, and NSAIDs.

In general, a unit dosage form will comprise in the range of from about 1 g to about 10 g of the polysaccharide, more usually from about 1.5 g to about 5 g of the polysaccharide, and most usually from about 2 g to about 4 g of the polysaccharide.

It is preferred that the unit dosage form is administered before consumption of a meal, in particular from 90-20 minutes before the meal, preferably from 60-30 minutes before the meal.

The unit dosage form is preferably administered together with an amount of liquid. This can be water, juice or any other beverage. Such amounts of liquid usually range from 100-500 mL. The appropriate dose should be taken at once with the liquid preferably 60-30 minutes before the meal. Next to its effect in reducing the symptoms of heartburn or GERD, it might create a sense of fullness, leading to a reduced amount of food intake during the meal. The actual dose an individual needs can be adjusted as a response to the amount of food about to be taken or the individual's sensitivity to particular foods, beverages or spices. The dose prior to dinner can by higher compared to the dose prior to a light lunch. Spicy foods might require a higher dose then less spicy foods.

In another aspect, the present invention is directed to a method for reducing the symptoms of heartburn and/or gastro-oesophageal reflux disease comprising administering an effective amount of a water-soluble branched or linear polysaccharide, wherein said polysaccharide has a hydration rate, measured by the viscosity development curve over time as measured during a 120 min period in a 1 wt.% solution hydrated at 60°C at 500 rpm in a Rapid Visco-analyser, reaching plateau viscosity within at most 20 minutes, and wherein said polysaccharide maintains at least 80 % of its number average molecular weight when exposed to an aqueous solution with a pH of 2-3 at 37 °C for at least 6 hours, and wherein the apparent viscosity of a 1 wt.% solution of said polysaccharide in water is at least 500 mPa·s (cps) at 20 °C and shear rates up to 60 s⁻¹ as measured by a Haake Rotoviscometer. Although the invention envisages a therapeutic treatment of heartburn and/or gastro-oesophageal reflux disease, i.e. a treatment which commences after symptoms have been observed, a major focus of the invention lies with a prophylactic treatment, i.e. a treatment which commences before symptoms have been observed, in order to prevent any discomfort in a subject that might be prone to heartburn and/or gastro-oesophageal reflux disease. When used prophylactically, a pharmaceutical preparation according to the invention is preferably administered prior to the intake of food.

The polysaccharide used in the method for reducing the symptoms of heartburn and/or gastro-oesophageal reflux disease preferably has a main chain which is composed for more than 50 wt.% of units that are chosen from the group consisting of glucose, galactose, mannose, glucuronic acid, galacturonic acid, and mannuronic acid.

In a further aspect, the invention encompasses the use of a pharmaceutical preparation as described above for the preparation of a medicament for treating heartburn and/or gastro-oesophageal reflux disease.

The pharmaceutical preparation of the invention may also be used to reduce temporary acid reflux symptoms due to pregnancy and to delay or prevent the onset and development of acid-peptic disorders in general, such as gastric and oesophageal ulcers, and/or conditions due to other damages to gastric mucosa and epithelium, such as gastritis.

The invention will now be elucidated by way of the following examples.

### Example 1

Fenugreek seeds were tempered in hot water, and dried again to regulate the water content in the seeds. The seeds were thereafter run through a flaking mill. The flakes were sieved to remove the smaller particles. The procedure of processing by flake milling and sieving was repeated to increase the level of galactomannan in the end product. The final product is milled into a homogeneous fine powder.

### Example 2

Fenugreek seeds were tempered in hot water, and dried again to regulate the water content in the seeds. The seeds were thereafter run through a flaking mill. The flakes were sieved to remove the smaller particles. The procedure of processing by flaking milling and sieving was repeated to increase the level of galactomannan in the end product. The flakes were treated with an ethanol/water-mixture (approximately 95 wt.%) to remove residual oils. The flakes were dried after recovery of the solvent, and milled into a homogeneous fine powder.

### Example 3

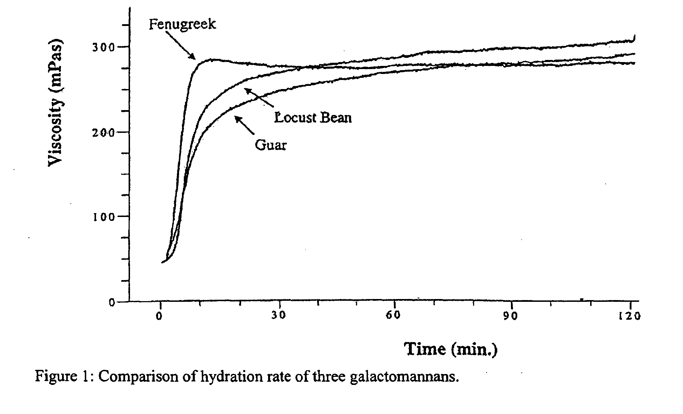

Comparison of the hydration rate of three galactomannan solutions as measured by a Rapid Visco-analyser (RVA). 1 % w/v galactomannan, containing 2 % v/v ethanol, was hydrated at 60 °C. To do so a sample of soluble fibre is pre-wetted with ethanol. The ethanol/gum ratio is 5ml /1g. Water is added to the pre-wetted sample in the ratio of approximately 16 / 1. The sample is shaken vigorously, before insertion into the RVA holder. The apparent viscosity was measured as a function of time in a RVA set to spin at 500 rpm during 120 min. The results are shown in Figure 1.

### Example 4

In a person diagnosed with *Hiatus hernia,* acid reflux has become a chronic problem. This individual took 6-8 g per day fenugreek fibre during 2 years in the form of capsules 30-60 minutes before a meal, together with sufficient water (at least 200 ml). The daily dose was divided into 3 portions, taken before breakfast, lunch and diner, respectively. Fenugreek fibre was rated to be 80-90 % effective in preventing reflux symptoms.

### Example 5

In a person diagnosed with *Hiatus hernia,* acid reflux has become a chronic problem. He was treated with various stomach acid reducing drugs, proton pump inhibitors and, over the counter, antacids. These treatments were only partially effective, leaving this individual with regular heartburns and occasional attacks of severe chest pain form acid reflux. This individual took:
2.4 - 3.2 g of fenugreek fibre before regular meals;
1.6 - 2.4 g of fenugreek fibre before smaller, bland meals; and
3.2 - 4.8 g of fenugreek fibre before spicy and/or rich meals.

The fibre was always taken together with a sufficient amount of water (at least 200 ml) during 2 years. The final result was that all drugs to treat his condition could be eliminated. The need for antacids was cut back to approximately 20 % of the dose needed at time zero.

## Claims

1. Pharmaceutical preparation comprising an effective amount of water-soluble branched or linear polysaccharide, wherein said polysaccharide has a hydration rate, measured by the viscosity development curve over time as measured during a 120 min period in a 1 wt.% solution hydrated at 60°C at 500 rpm in a Rapid Visco-analyser, reaching maximum viscosity within at most 20 minutes, and wherein said polysaccharide maintains at least 80 % of its number average molecular weight when exposed to an aqueous solution with a pH of 2-3 at 37 °C for at least 6 hours, and wherein the apparent viscosity of a 1 wt.% solution of said polysaccharide in water is at least 500 mPa·s (cps) at 20 °C and shear rates up to 60 s⁻¹ as measured by a Haake Rotoviscometer, wherein the polysaccharide is a galactomannan obtained from fenugreek seeds,

2. Preparation according to claim 1, wherein the polysaccharide is a fenugreek galactomannan with a degree of substitution of 0.25-1.1.

3. Preparation according to any of the previous claims, wherein the apparent viscosity of a 1.5 wt.% solution of the polysaccharide in water at 20 °C is at least 1500 mPa·s (cps) as measured by a Haake Rotoviscometer.

4. Preparation according any of the previous claims in a delivery form, by which the preparation is protected from early exposure to moisture in the mouth and oesophagus.

5. Preparation according claim 4, wherein said delivery form is a capsule, coated tablet or coated pill.

6. Preparation according to any of the previous claims, wherein said preparation further comprises a pharmaceutically acceptable excipient chosen from the classes of diluents, disintegrants, granulating agents, lubricants, binders, thickeners, flavouring agents, colouring agents, preservatives, fillers, sweeteners, antioxidants, and coating materials.

7. Fenugreek galactomannan as defined in claims 1 or 2 for use in the treatment of heartburn and/or gastro-oesophageal reflux disease.

8. Fenugreek galactomannan according to claim 7, wherein said treatment comprises administering said polysaccharide 90-20 minutes, preferably 60-30 minutes, before the consumption of a meal.

9. Fenugreek galactomannan according to claim 7 or 8, wherein said treatment further comprises administering an amount of liquid ranging from 100-500 mL.

10. Use of a fenugreek galactomannan as defined in claim 1 or 2 for the manufacture of a medicament for delaying or preventing the onset and development of acid-peptic disorders and/or conditions due to other damages to gastric mucosa and epithelium.

11. Use of fenugreek galactomannan as defined in claim 1 or 2 for the manufacture of a medicament for reducing temporary acid reflux symptoms due to pregnancy.

## Patentansprüche

1. Pharmazeutische Zubereitung mit einer wirksamen Menge an wasserlöslichem verzweigten oder linearen Polysaccharid, wobei das Polysaccharid eine Hydrationsrate hat, gemessen durch die Viskositätsentwicklungskurve über der Zeit bei Messung während einer Periode von 120 Min. in einer bei 60°C mit 500 u/min. hydrierten Lösung von 1 Gew.-% in einer Rapid-Visco-Analysevorrichtung, bei der die maximale Viskosität innerhalb höchstens 20 Minuten erreicht wird, und wobei das Polysaccharid mindestens 80% seines zahlengemittelten Molekulargewichts beibehält, wenn es mindestens 6 Stunden einer wässrigen Lösung mit einem pH-Wert von 2-3 bei 37°C ausgesetzt wird, und wobei bei Messung mit einem Haake-Rotoviscometer die scheinbare Viskosität einer Lösung von 1 Gew.-% des Polysaccharids in Wasser bei 20°C mindestens 500 mPa·s (cpa) und die Scherraten bis zu 60 s⁻¹ betragen, wobei das Polysaccharid ein aus Bockshornklee erhaltenes Galactomannan ist.

2. Zubereitung nach Anspruch 1, bei der das Polysaccharid ein Bockshornklee-Galactomannan mit einem Substitutionsgrad von 0,25-1,1 ist.

3. Zubereitung nach einem der vorherigen Ansprüche, bei der bei Messung mit einem Haake-Rotoviscometer die scheinbare Viskosität einer Lösung von 1,5 Gew.-% des Polysaccharids in Wasser bei 20°C mindestens 1500 mPa·s (cpa) beträgt.

4. Zubereitung nach einem der vorherigen Ansprüche in Verabreichungsform, wobei die Zubereitung davor geschützt ist, im Mund und in der Speiseröhre frühzeitig Feuchtigkeit ausgesetzt zu werden.

5. Zubereitung nach Anspruch 4, bei der die Verabreichungsform in einer Kapsel, einer beschichteten Tablette oder einer beschichteten Pille besteht.

6. Zubereitung nach einem der vorherigen Ansprüche, wobei die Zubereitung ferner einen pharmazeutisch akzeptablen Hilfsstoff aufweist, der aus den Klassen von Verdünnungsmitteln, Abbaumittel, Granuliermitteln, Schmiermitteln, Bindemitteln, Verdickungsmitteln, Geschmacksstoffen, Färbemitteln, Konservierungsmitteln, Füllstoffen, Süßstoffen, Antoxidantien und Beschichtungsmateralien gewählt ist.

7. Bockshornklee-Galactomannan nach Anspruch 1 oder 2 zur Verwendung bei der Behandlung von Sodbrennen und/oder gastroösophagealer Reflux-Krankheiten.

8. Bockshornklee-Galactomannan nach Anspruch 7, bei der die Behandlung die Verabreichung des Polysaccharids 90-20 Minuten, vorzugsweise 60-30 Minuten vor der Einnahme einer Mahlzeit umfaßt.

9. Bockshornklee-Galactomannan nach Anspruch 7 oder 8, bei der die Behandlung ferner die Verabreichung einer Flüssigkeitsmenge von 100-500 mL umfasst.

10. Verwendung eines Bockshornklee-Galactomannans nach Anspruch 1 oder 2 zur Herstellung eines Medikaments zum Verzögern oder Verhindern des Beginns und der Entwicklung säuerungs-peptischer Störungen und/oder Zustände aufgrund anderer Schädigungen von Magenschleimhaut und -epithel.

11. Verwendung eines Bockshornklee-Galactomannans nach Anspruch 1 oder 2 zur Herstellung eines Medikaments zum Verzögern vorübergehender Säurereflux-Symptome aufgrund von Schwangerschaft.

## Revendications

1. Préparation pharmaceutique comprenant une quantité efficace d'un polysaccharide linéaire ou ramifié soluble dans l'eau, dans laquelle ledit polysaccharide présente une vitesse d'hydratation, mesurée par la courbe de développement de la viscosité au cours du temps comme mesurée sur une durée de 120 min dans une solution à 1% en masse hydratée à 60°C à 500 tr/min dans un dispositif Rapid Visco-analyser, atteignant une viscosité maximale en au plus 20 min, et dans laquelle ledit polysaccharide conserve au moins 80 % de sa masse moléculaire moyenne en nombre lorsqu'il est exposé à une solution aqueuse avec un pH de 2-3 à 37°C pendant au moins 6 h, et dans laquelle la viscosité apparente d'une solution à 1 % en masse dudit polysaccharide dans l'eau est d'au moins 500 mPa.s (cps) à 20°C et à des vitesses de cisaillement allant jusqu'à 60 s⁻¹ comme mesurée par un roto-viscosimètre Haake, dans laquelle le polysaccharide est un galactomannane obtenu à partir de graines de fenugrec.

2. Préparation selon la revendication 1, dans laquelle le polysaccharide est un galactomannane de fenugrec avec un degré de substitution de 0,25-1,1.

3. Préparation selon l'une quelconque des revendications précédentes, dans laquelle la viscosité apparente d'une solution à 1,5% en masse du polysaccharide dans l'eau à 20°C est d'au moins 1 500 MPa·s (cps) comme mesurée par un roto-viscosimètre Haake.

4. Préparation selon l'une quelconque des revendications précédentes dans une forme d'administration par laquelle la préparation est protégée d'une exposition prématurée à l'humidité dans la bouche et dans l'oesophage.

5. Préparation selon la revendication 4, dans laquelle ladite forme d'administration est une capsule, un comprimé enrobé ou une pilule enrobée.

6. Préparation selon l'une quelconque des revendications précédentes, dans laquelle ladite préparation comprend de plus un excipient pharmaceutiquement acceptable choisi dans les classes des diluants, désintégrants, agents de granulation, lubrifiants, liants, épaississants, agents aromatisants, agents colorantes, conservateurs, charges, édulcorants, antioxydants, et matériaux d'enrobage.

7. Galactomannane de fenugrec selon la revendication 1 ou 2 pour une utilisant dans le traitement d'une maladie de pyrosis et/ou de reflux gastro-oesophagien.

8. Galactomannane de fenugrec selon la revendication 7, dans lequel ledit tracement comprend l'administration dudit polysaccharide 90-20 minutes, de préférence 60-80 minutes, avant la consommation d'un repas.

9. Galactomannane de fenugrec selon la revendication 7 ou 8, dans lequel redit traitement comprend de plus l'administration d'une quantité de liquide comprise entre 100 et 500 ml.

10. Utilisation d'un galactomannane de fenugrec selon la revendication 1 ou 2 pour la fabrication d'un medicament pour retarder le ou prévenir du démarrage et développement de troubles acide-peptiques et/ou d'états dus à d'autres détériorations de la muqueuse gastrique et de l'épithélium,

11. Utilisation d'un galactomannane de fenugrec selon la revendication 1 ou 2 pour la fabrication d'un médicament destiné à réduire les symptômes temporaires de reflux acide dus à la grossesse.
